# EUROPÄISCHE PATENTANMELDUNG

(11) **EP 4 691 502 A1**
(43) Veröffentlichungstag der Anmeldung: **11.02.2026**
(21) Anmeldenummer: 25182120.3
(22) Anmeldetag: 11.06.2025
(51) Int. Cl.: A61L 9/012, A61L 9/013, A61L 9/02, A61L 9/05, A61H 33/06, A61K 36/23, A61K 36/53, A61K 36/534, A61K 36/61, A61K 36/67, A61K 36/752, A61P 39/00, A61L 101/34, A61L 101/46

(54) **KAPSEL, VERFAHREN ZUM HERSTELLEN EINER KAPSEL, VERFAHREN ZUM HERSTELLEN EI-NES ÄTHERISCHEN AEROSOLS UND BLISTER MIT DER KAPSEL**

(30) Priorität: 05.08.2024 DE 202024104401 U
(71) Anmelder: Reindl, Christian, 92648 Vohenstrauß (DE)
(72) Erfinder: Reindl, Christian, 92648 Vohenstrauß (DE)
(74) Vertreter: Knoke, Isabel Yvonne

(57) **Zusammenfassung**

Die Erfindung betrifft eine Kapsel (2), umfassend
- eine geschlossene Hülle (4),
- eine in der Hülle (4) aufgenommene Flüssigkeit (20),

wobei die Flüssigkeit (20) ein duftendes ätherisches Öl und ein geruchsneutralen Alkohol, insbesondere 3-Methoxy-3-methylbutanol, enthält, wobei der Anteil des geruchsneutralen Alkohols, insbesondere 3-Methoxy-3-methylbutanol, zumindest 30 Gew.% beträgt,
wobei die geschlossene Hülle (4) aus einer wasserlöslichen und gegenüber der Flüssigkeit (20) beständigen Folie hergestellt ist.

Weiterhin betrifft die Erfindung ein Verfahren zur Herstellung des Aerosols (50) mit der Kapsel (2) und ein Verfahren zur Herstellung der Kapsel (2).

## Beschreibung

Die Erfindung betrifft eine Kapsel, ein Verfahren zum Herstellen einer Kapsel, ein Verfahren zum Herstellen eines ätherischen Aerosols und ein Blister mit der Kapsel.

Für Saunaanwendungen ist es bekannt, ätherische Öle mit Wasser zu vermischen und die Mischung auf den heißem Saunasteinen zu verdampfen, so dass duftende Aerosole entstehen. Diese Aerosole können zum einen ein schönes Dufterlebnis bewirken, zum anderen können einige Duftstoffe auch eine gesundheitsfördernde oder Wohlbefinden verbessernde Wirkung aufweisen. Zur Aufbewahrung solcher ätherischen Öle und Duftstoffe müssen diese in verschlossenen Behältnissen aufbewahrt werden, um ein Verflüchtigen der Duftstoffe zu minimieren. Bekannterweise werden solche Öle beispielsweise in Glasflaschen aufbewahrt und können dann vom Nutzer dosiert werden.

Aus der DE 29 43 662 A1 ist ein Verfahren und eine Vorrichtung zum Herstellen eines künstlichen Aerosols mit Anteilen von ätherischen Ölen bekannt. Die daraus bekannte Weichgelantinekapsel weist eine Füllung aus einem flüssigem ätherischen Öl auf. Der Nachteil der bekannten Weichgelantinekapsel zur Herstellung eines künstlichen Aerosols mit Anteilen von ätherischen Ölen besteht darin, dass Weichgelantine sich nicht rückstandsfrei auflöst, sondern sich bei der Verdampfung auf den Saunasteinen Rückstände bilden, die zu einer Verschmutzung der Steine führen.

Die Aufgabe der Erfindung ist es, eine Kapsel für Saunaanwendungen anzugeben, welche umweltfreundlich sind und bei der Auflösung bzw. Verdampfung keine zu entsorgenden Rückstände bilden.

Diese Aufgabe wird durch den Gegenstand des Anspruchs 1 gelöst. Zweckdienliche Ausgestaltung ergeben sich aus den abhängigen Ansprüchen.

Die erfindungsgemäße Kapsel umfasst
- eine geschlossene, insbesondere flexible, Hülle,
- eine in der Hülle aufgenommene Flüssigkeit,

wobei die Flüssigkeit ein duftendes ätherisches Öl und einen geruchsneutralen Alkohol, insbesondere 3-Methoxy-3-methylbutanol, enthält, wobei der Anteil des geruchsneutralen Alkohols, insbesondere 3-Methoxy-3-methylbutanol, zumindest 30 Gew.% beträgt,
wobei die geschlossene Hülle aus einer wasserlöslichen Folie hergestellt ist. Die Flüssigkeit greift die Hülle nicht an. Somit ist die geschlossene Hülle gegenüber der Flüssigkeit beständig.

Der geruchsneutrale Alkohol hat insbesondere einen Siedepunkt oberhalb des Siedepunkts von Wassern insbesondere oberhalb von 150°C. 3-Methoxy-3-methylbutanol ist ein Alkohol mit einer geringen Verdunstungsgeschwindigkeit der für eine gleichmäßigere Duftfreisetzung sorgt und geruchsneutral. Der Siedepunkt von 3-Methoxy-3-methylbutanol liegt bei 173°C.

Zweckmäßigerweise ist die Folie aus einem der folgenden Materialien hergestellt: Polyvinylalkohol (PVA), teilhydrolysiertem Polyvinylalkohol, Gelatine, Agar-Agar, Pektin, Carrageen, Chitosan, Pullulan, Hydroxypropylmethylcellulose, Alginat. Insbesondere ist die Folie aus teilhydrolysiertem Polyvinylalkohol hergestellt.

Eine Folienstärke bzw. Wandstärke der geschlossenen Hülle kann beispielsweise im Bereich von 0,01 mm bis 0,1 mm liegen, insbesondere 0,03mm bis 0,04mm, beispielsweise 0,35mm oder kann im Bereich von 0,05 mm bis 0,09 mm liegen.

In Ausgestaltung enthält das ätherische Öl ein oder mehrere Öle aus Eukalyptusöl, Pfefferminzöl, Rosmarinöl, Zitronenöl, Grapefruitöl, Orangenöl, Lavendelöl, Thymianöl, Kamillenöl, Tannenöl oder das ätherische Öl besteht aus einem der vorgenannten Öle. Das ätherische Öl ist insbesondere ein natürliches ätherisches Öl oder eine Mischung natürlicher ätherischer Öle.

In einer Ausgestaltung ist die geschlossene Hülle durch zwei Folienabschnitte gebildet, welche entlang ihrer jeweiligen Ränder miteinander, insbesondere durch Schweißen, fest verbunden sind. Zweckmäßigerweise ist das Schweißen ein thermisches Schweißen bei einer Temperatur von 90°C bis 110°C.

In einer alternativen Ausgestaltung ist die geschlossene Hülle aus einem Folienschlauchabschnitt gebildet, welcher an den offenen Enden, insbesondere durch Schweißen, verschlossen wird. In einer weiteren alternativen Ausgestaltung ist die geschlossene Hülle aus einem gefalteten Folienabschnitt gebildet, welcher dann an den offenen, insbesondere 3 offenen Seiten verschweißt ist.

In Ausgestaltung weist die Kapsel in Aufsicht eine im Wesentlichen runde oder quadratische Form auf. Eine Seitenkante kann bei einer quadratischen Form beispielsweise im Bereich von 2 bis 4 cm liegen. Beispielsweise weist die Kapsel eine Kantenlänge von 3 cm auf. Alternativ kann die Kapsel beispielsweise einen Durchmesser im Bereich von 2 bis 5 cm aufweisen. Die Kapsel kann beispielsweise für eine Füllmenge von 8 ml ausgelegt sein.

In einer Ausgestaltung enthält die Flüssigkeit 40 Gew.-% bis 55 Gew.% 3-Methoxy-3-methylbutanol. Die Flüssigkeit kann insbesondere 45 Gew.-% bis 50 Gew.% 3-Methoxy-3-methylbutanol enthalten, beispielsweise kann die Flüssigkeit 47 Gew.-% bis 48 Gew.% 3-Methoxy-3-methylbutanol enthalten. Der Anteil an 3-Methoxy-3-methylbutanol kann die Länge der Duftwahrnehmung bei einer unten beschriebenen Herstellen eines ätherischen Aerosols zur Anwendung in einer Sauna, das heißt bei einer Anwendung während eines Saunagangs wesentlich beeinflussen. Ein Anteil von 47 Gew.-% bis 48 Gew.% 3-Methoxy-3-methylbutanol kann gegenüber niedrigeren Anteilen die Duftwahrnehmung auf einen Zeitraum von ca. 12 Minuten ausdehnen, was im Bereich eines typischen Saunagangs liegt.

In einer Ausgestaltung weist die Flüssigkeit ein Mischungsverhältnis von 45 Teilen ätherisches Öl zu 35 Teilen 3-Methoxy-3-methylbutanol auf. Bei einer Füllmenge von 8 ml entspricht dies 4,5 ml ätherischem Öl und 3,5 ml 3-Methoxy-3-methylbutanol.

Der Flüssigkeit können ein oder mehrere funktionale Zusatzstoffe zugesetzt sein, welches (welche) zumindest eine pflegende, belebende oder wohltuende Eigenschaft aufweist (aufweisen), und welches (welche) insbesondere durch die Haut und/oder die Atemwege, z.B. über die Lunge, aufgenommen werden kann (können).

Der Flüssigkeit können weiterhin eine oder mehrere der folgenden Substanzen, insbesondere als funktionale Zusatzstoffe, hinzugesetzt sein: Magnesiumsalze, Koffein, Arnikaextrakt, Menthol, Panthenol, Triethylcitrat, pflanzliches Squalan, Linalool, Vetiveröl, Zinkricinoleat, Eucalyptol.

Diese können eine belebende, gesundheitsfördernde oder Wohlbefinden verbessernde Wirkung für den Nutzer bei Anwendung als Aerosol in einer Sauna haben oder eine solche unterstützen.

Die vorgenannten Zusatzstoffe bzw. Substanzen können in der Flüssigkeit löslich oder unlöslich sein. Im letzteren Fall bildet sich insbesondere eine Dispersion oder Emulsion.

Das erfindungsgemäße Verfahren zum Herstellen einer oben beschriebenen Kapsel, umfasst
- Bereitstellen eines ersten Folienabschnitts,
- Tiefziehen des ersten Folienabschnitts,
- Befüllen des tiefgezogenen ersten Folienabschnitts mit einer Flüssigkeit, die ein duftendes ätherisches Öl und einen geruchsneutralen Alkohol, insbesondere 3-Methoxy-3-methylbutanol, enthält,
- Abdecken des befüllten tiefgezogenen ersten Folienabschnitts mit einem zweiten Folienabschnitt und
- Verschweißen der Ränder der Folienabschnitte so miteinander, dass eine geschlossene Hülle entsteht. Der erste Folienabschnitt und der zweite Folienabschnitt sind dabei aus wasserlöslicher und gegenüber der oben beschriebenen Flüssigkeit beständiger Folie. Der Flüssigkeit können die oben genannten Zusatzstoffe oder Substanzen hinzugesetzt sein.

Das alternative erfindungsgemäße Verfahren zum Herstellen einer oben beschriebenen Kapsel, umfasst
- Bereitstellen eines ersten Folienabschnitts,
- Falten, insbesondere hälftiges Falten, des ersten Folienabschnitts,
- Verschweißen von zumindest zwei offenen Randabschnitten des gefalteten ersten Folienabschnitts, so dass eine einseitig offene Tüte entsteht,
- Befüllen der Tüte mit einer Flüssigkeit, die ein duftendes ätherisches Öl und einen geruchsneutralen Alkohol, insbesondere 3-Methoxy-3-methylbutanol, enthält,
- Verschweißen der noch offenen Randabschnitte der Folienabschnitte so miteinander, dass eine geschlossene Hülle entsteht. Der erste Folienabschnitt ist dabei aus wasserlöslicher und gegenüber der oben beschriebenen Flüssigkeit beständiger Folie. Der Flüssigkeit können die oben genannten Zusatzstoffe oder Substanzen hinzugesetzt sein.

Insbesondere entstehen bei hälftigen Falten eines rechteckigen ersten Folienabschnitts drei offene Randabschnitte, von denen zunächst zwei durch Schweißen geschlossen werden und nach dem Befüllen die verbleibenden dritten Randabschnitte durch Schweißen geschlossen werden.

Das erfindungsgemäße Verfahren zum Herstellen eines ätherischen Aerosols zur Anwendung in einer Sauna, umfasst:
- Bereitstellen eines Gefäßes mit einer vorbestimmten Menge Wasser,
- Einbringen einer oder mehrerer oben beschriebener Kapseln in das Gefäß mit der vorbestimmten Menge Wasser,
- Auflösen der einen oder mehreren Kapseln in der vorbestimmten Menge Wasser, so dass sich die Hülle vollständig auflöst und die in den Kapseln enthaltene Flüssigkeit sich mit dem Wasser mischt,
- Aufbringen des Flüssigkeits-Wasser-Gemisches auf heiße Saunasteine zur Erzeugung eines Aerosols.

Das Wasser hat zweckmäßigerweise eine Temperatur von 15-40°C, insbesondere 20-40°C.

Zweckmäßigerweise ist die vorbestimmte Menge Wasser 1-2 Liter Wasser, insbesondere 1 Liter oder 1,5 Liter, und zweckmäßigerweise wird genau eine Kapsel in das Wasser eingebracht.

In einer Ausgestaltung liegt eine Auflösedauer der Hülle im Bereich von 20s bis 180s, insbesondere liegt die Auflösedauer im Bereich von 30s bis 40s oder im Bereich von 100s bis 120s, bevorzugt bei 120s.

Der erfindungsgemäße Blister enthält eine oder mehrere oben beschriebene Kapseln. Ein solcher Blister umfasst eine, insbesondere transparente, nicht-wasserlösliche tiefgezogene Folie, mit einer Mulde, in die die Kapsel gelegt ist, wobei die Mulde durch eine weitere nicht-wasserlösliche Folie abgedeckt und verschlossen ist. Die nicht-wasserlösliche tiefgezogene Folie und die weitere nicht-wasserlösliche Folie können aus unterschiedlichen Materialien hergestellt sein. Insbesondere kann die weitere Folie dazu ausgelegt sein, zur Entnahme der Kapsel zu zerreißen, wenn die Kapsel von der Seite der nicht-wasserlöslichen tiefgezogenen Folie gegen die weitere Folie gedrückt wird.

Die Erfindung wird nachstehend auch hinsichtlich weiterer Merkmale und Vorteile anhand der Beschreibung von Ausführungsbeispielen und unter Bezugnahme auf die beiliegenden Zeichnungen näher erläutert. Es zeigen jeweils in einer schematischen Prinzipskizze:
- Fig. 1: erste Ausgestaltung der Kapsel,
- Fig. 2: Schnittansicht der ersten Ausgestaltung,
- Fig. 3a: Aufsicht auf die Kapsel aus Fig. 1,
- Fig. 3b: Aufsicht auf eine Kapsel einer zweiten Ausgestaltung,
- Fig. 4: Herstellung des Aerosols.

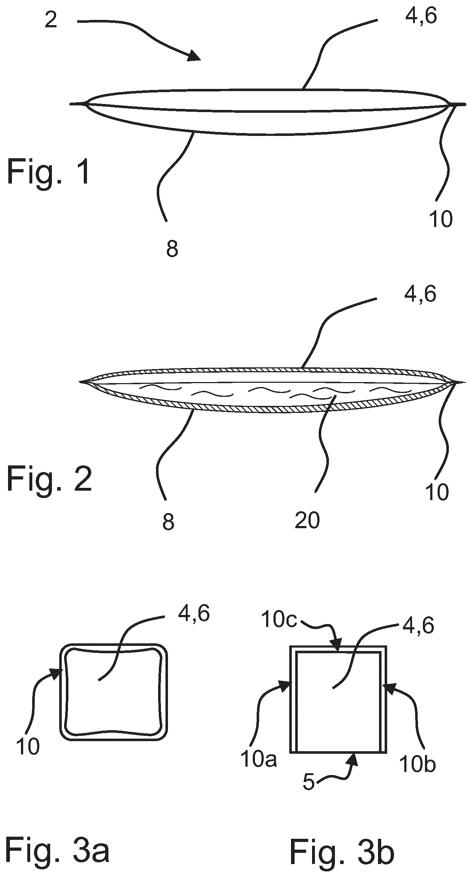

Fig. 1 zeigt eine Kapsel 2, welche eine geschlossene Hülle 4 umfasst. Die Hülle 4 ist hier aus einem ersten Folienabschnitt 6 und einem zweiten Folienabschnitt 8 gebildet, welche an ihren Rändern 10 miteinander so verbunden sind, dass in der Hülle 4 aufgenommene Flüssigkeit nicht entweichen kann. Der erste Folienabschnitt 6 und der zweite Folienabschnitt 8 sind aus einem wasserlöslichen Material gebildet. Die Folienabschnitte sind stabil gegenüber der Flüssigkeit. Die Kapsel 2 kann entweder relativ flach als auch nahe zu kubisch, mit einer Höhe die ähnlich der Länge und Breite ist, sein.

Fig. 2 zeigt eine Schnittansicht durch die Kapsel 2. Zwischen dem ersten Folienabschnitt 6 und dem zweiten Folienabschnitt 8 ist eine Flüssigkeit 20 aufgenommen. Der erste und der zweite Folienabschnitt 6, 8 können jeweils eine Wandstärke wie oben beschrieben aufweisen. In der Kapsel 2 kann zusätzlich zur Flüssigkeit 20 weiterhin ein Luftvolumen eingeschlossen sein, welches aber zweckmäßigerweise kleiner als das in der Hülle 4 enthaltene Flüssigkeitsvolumen ist. Die Flüssigkeit 20 umfasst ein duftendes ätherisches Öl.

Fig. 3a zeigt eine Aufsicht auf die Kapsel 2 nach der ersten Ausgestaltung. Der erste Folienabschnitt 6 der Hülle 4 ist hier dem Betrachter zugewandt. Umlaufend ist ein Rand 10 mit dem in Sichtrichtung dahinterliegenden zweiten Folienabschnitt 8 verbunden.

Fig. 3b zeigt eine alternative Ausgestaltung der Kapsel, bei dem die Hülle 4 aus einem gefalteten ersten Folienabschnitt 6 gebildet ist. Die Faltkante ist hier mit dem Bezugszeichen 5 markiert. Der erste Folienabschnitt 6 wird zunächst an zwei der drei Randabschnitte 10a,b, c, z.B. 10a und 10b, verschweißt, dann befüllt und nach dem Befüllen kann der letzte der drei Randabschnitte 10a,b, c, im Beispiel 10c, verschweißt werden.

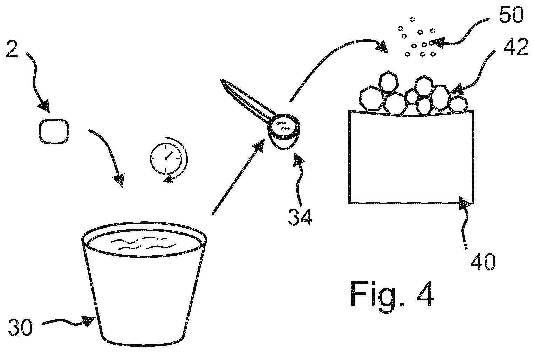

Fig. 4 zeigt schematisch das Verfahren zum Herstellen eines Aerosols 50. Dazu wird zunächst ein Gefäß 30, z.B. ein Eimer, mit einer vorbestimmten Menge Wasser, z. B. 1 Liter, bereitgestellt. Eine die Flüssigkeit 20 enthaltende Kapsel 2 wird in das Gefäß 30 mit dem Wasser gegeben. Die Hülle 4 der Kapsel 2 löst sich nun in einer vorgegebenen Zeit in der vorbestimmten Menge Wasser vollständig auf und die in den Kapseln 2 enthaltene Flüssigkeit 20 mischt sich mit dem Wasser. Mit einer Schöpfkelle 34 kann nun beispielsweise Flüssigkeits-Wasser-Gemisch aus dem Gefäß 30 entnommen werden und auf heiße Saunasteine aufgebracht werden. Durch die Wärme der Saunasteine wird ein Aerosol 50 erzeugt. Dabei verdampft das Gemisch so, dass keine Rückstände der Hülle 4 auf den Saunasteinen 42 zurückbleiben.

### Bezugszeichenliste

- 2: Kapsel
- 4: Hülle
- 5: Faltkante
- 6: erster Folienabschnitt
- 8: zweiter Folienabschnitt
- 10: Rand
- 10a,b,c: Randabschnitte
- 20: Flüssigkeit
- 30: Gefäß
- 34: Schöpfkelle
- 40: Saunaofen
- 42: Saunasteine
- 50: Aerosol

## Patentansprüche

1. Kapsel (2), umfassend
- eine geschlossene Hülle (4),
- eine in der Hülle (4) aufgenommene Flüssigkeit (20),
wobei die Flüssigkeit (20) ein duftendes ätherisches Öl und einen geruchsneutralen Alkohol, insbesondere 3-Methoxy-3-methylbutanol, enthält, wobei der Anteil des geruchsneutralen Alkohols, insbesondere 3-Methoxy-3-methylbutanol, zumindest 30 Gew.% beträgt,
wobei die geschlossene Hülle (4) aus einer wasserlöslichen und gegenüber der Flüssigkeit (20) beständigen Folie hergestellt ist.

2. Kapsel (2) nach Anspruch 1,
wobei die Folie aus einem der folgenden Materialien hergestellt ist:
Polyvinylalkohol (PVA), teilhydrolysiertem Polyvinylalkohol, Gelatine, Agar-Agar, Pektin, Carrageen, Chitosan, Pullulan, Hydroxypropylmethylcellulose, Alginat, insbesondere wobei die Folie aus teilhydrolysiertem Polyvinylalkohol hergestellt ist.

3. Kapsel (2) nach Anspruch 1 oder 2,
wobei das ätherische Öl ein oder mehrere Öle aus Eukalyptusöl, Pfefferminzöl, Rosmarinöl, Zitronenöl, Grapefruitöl, Orangenöl, Lavendelöl, Thymianöl, Kamillenöl, Tannenöl enthält oder daraus besteht.

4. Kapsel (2) nach einem der vorstehenden Ansprüche,
wobei die geschlossene Hülle (4) durch zwei Folienabschnitte (6,8) gebildet ist, welche entlang ihrer jeweiligen Ränder (10) miteinander, insbesondere durch Schweißen, insbesondere durch thermisches Schweißen bei 90°C bis 110 °C , fest verbunden sind, oder
wobei die geschlossene Hülle (4) aus einem gefalteten Folienabschnitt (6) gebildet ist.

5. Kapsel (2) nach einem der vorstehenden Ansprüche, wobei die geschlossene Hülle eine Wandstärke im Bereich von 0,01 mm bis 0,1 mm aufweist, insbesondere 0,03mm bis 0,04mm, beispielsweise 0,35mm oder im Bereich von 0,05 mm bis 0,09 mm aufweist.

6. Kapsel (2) nach einem der vorstehenden Ansprüche, wobei die Flüssigkeit (20) 40 Gew.-% bis 55 Gew.% 3-Methoxy-3-methylbutanol enthält, wobei die Flüssigkeit (20) insbesondere 45 Gew.-% bis 50 Gew.% 3-Methoxy-3-methylbutanol enthält, wobei die Flüssigkeit (20) 47 Gew.-% bis 48 Gew.% 3-Methoxy-3-methylbutanol enthält.

7. Kapsel (2) nach einem der vorstehenden Ansprüche, wobei die Flüssigkeit ein Mischungsverhältnis von 45 Teilen ätherisches Öl zu 35 Teilen 3-Methoxy-3-methylbutanol aufweist.

8. Kapsel (2) nach einem der vorstehenden Ansprüche, wobei der Flüssigkeit (20) weiterhin ein oder mehrere funktionale Zusatzstoffe zugesetzt sind, welches zumindest eine pflegende, belebende oder wohltuende Eigenschaft aufweist, und welches insbesondere durch die Haut und/oder die Atemwege aufgenommen werden kann.

9. Kapsel (2) nach einem der vorstehenden Ansprüche, wobei der Flüssigkeit eine oder mehrere der folgenden Substanzen, insbesondere als funktionale Zusatzstoffe, hinzugesetzt sind: Magnesiumsalze, Koffein, Arnikaextrakt, Menthol, Panthenol, Triethylcitrat, pflanzliches Squalan, Linalool, Vetiveröl, Zinkricinoleat, Eucalyptol.

10. Verfahren zum Herstellen einer Kapsel (2) nach einem der vorstehenden Ansprüche, umfassend
- Bereitstellen eines ersten Folienabschnitts (6) aus wasserlöslicher und gegenüber einer Flüssigkeit (20) beständiger Folie,
- Tiefziehen des ersten Folienabschnitts (6),
- Befüllen des tiefgezogenen ersten Folienabschnitts (6) mit der Flüssigkeit (20), die ein duftendes ätherisches Öl und einen geruchsneutralen Alkohol, insbesondere 3-Methoxy-3-methylbutanol, enthält ,
- Abdecken des befüllten tiefgezogenen Folienabschnitts (6) mit einem zweiten Folienabschnitt (8) aus wasserlöslicher und gegenüber der Flüssigkeit (20) beständiger Folie und
- Verschweißen der Ränder (10) der Folienabschnitte (6, 8) miteinander, dass eine geschlossene Hülle (4) entsteht.

11. Verfahren zum Herstellen einer Kapsel (2) nach einem der Ansprüche 1-9, umfassend
- Bereitstellen eines ersten Folienabschnitts (6) aus wasserlöslicher und gegenüber einer Flüssigkeit (20) beständiger Folie,
- Falten des ersten Folienabschnitts (6),
- Verschweißen von zumindest zwei offenen Randabschnitten (10a, b) des gefalteten ersten Folienabschnitts (6), so dass eine einseitig offene Tüte entsteht,
- Befüllen der Tüte mit der Flüssigkeit (20), die ein duftendes ätherisches Öl und einen geruchsneutralen Alkohol, insbesondere 3-Methoxy-3-methylbutanol, enthält,
- Verschweißen der noch offenen Randabschnitte (10c) des gefalteten Folienabschnitts (6) so miteinander, dass eine geschlossene Hülle (4) entsteht.

12. Verfahren zum Herstellen eines ätherischen Aerosols (50) zur Anwendung in einer Sauna, umfassend:
- Bereitstellen eines Gefäßes (30) mit einer vorbestimmten Menge Wasser,
- Einbringen einer oder mehrerer Kapseln (2) nach einem der Ansprüche 1 bis 9 in das Gefäß (30) mit der vorbestimmten Menge Wasser,
- Auflösen der einen oder mehreren Kapseln (2) in der vorbestimmten Menge Wasser, so dass sich die Hülle (4) vollständig auflöst und die in den Kapseln enthaltene Flüssigkeit (20) sich mit dem Wasser mischt,
- Aufbringen des Flüssigkeits-Wasser-Gemisches auf heiße Saunasteine (42) zur Erzeugung des Aerosols (50).

13. Verfahren nach Anspruch 12, wobei die vorbestimmte Menge Wasser 1 bis 2 Liter ist, insbesondere 1 Liter oder insbesondere 1,5 Liter ist, und genau eine Kapsel (2) in das Wasser eingebracht wird.

14. Verfahren nach Anspruch 12 oder 13, wobei eine Auflösedauer der Hülle (4) im Bereich von 20s bis 180s liegt, insbesondere wobei die Auflösedauer im Bereich von 30s bis 40s liegt oder insbesondere wobei die Auflösedauer im Bereich von 100s bis 120s, bevorzugt bei 120s, liegt.

15. Blister mit einer oder mehreren Kapseln (2) nach einem der Ansprüche 1 bis 9.
